# EUROPÄISCHE PATENTSCHRIFT

(11) **EP 4 582 801 B1**
(45) Veröffentlichungstag und Bekanntmachung des Hinweises auf die Patenterteilung: **03.12.2025**
(21) Anmeldenummer: 24217515.6
(22) Anmeldetag: 04.12.2024
(51) Int. Cl.: G01N 33/08, A01K 43/00

(54) **EINRICHTUNG ZUR UNTERSUCHUNG VON BRUTEIERN**
DEVICE FOR EXAMINING BROODIANS
DISPOSITIF POUR EXAMINER DES OEUFS DE COUVAIN

(30) Priorität: 03.01.2024 DE 102024000143; 03.01.2024 DE 202024000107 U
(43) Veröffentlichungstag der Anmeldung: 09.07.2025
(73) Patentinhaber: EVONTA-Technology GmbH, 01109 Dresden (DE)
(72) Erfinder: Fischer, Björn, Dr., 09212 Limbach-Oberfrohna (DE); Meissner, Sven, Dr., 09618 Brand-Erbisdorf (DE)
(74) Vertreter: Kailuweit & Uhlemann Patentanwälte Partnerschaft mbB

(56) Entgegenhaltungen:
- EP-A1- 3 437 468
- DE-T2- 60 003 914
- US-A1- 2010 141 933

## Beschreibung

Die Erfindung betrifft eine Einrichtung zur Untersuchung von Bruteiern mit einer Transporteinrichtung, Lichtquellen und Kameras in Verbindung mit einem Datenverarbeitungssystem.

Eine frühzeitige Beurteilung von Bruteiern im Brutprozess ist insbesondere für die kommerzielle Geflügelproduktion von großer Bedeutung. Dabei ist unter anderem auch der Entwicklungszustand und eine Bestimmung des Geschlechts des Bruteies unter Beachtung des Tierschutzes von großem Interesse.

Durch die Druckschrift DE 10 2007 013 107 A1 ist ein Verfahren zur Bestimmung des Geschlechts von Vögeln offenbart, wobei ein DNA-relevantes Zellmaterial aus dem Schaft einer jungen Feder des Vogels untersucht wird. Ein durch Bestrahlung mit Licht entstehendes Spektrum wird mit Referenzspektren verglichen, so dass eine Geschlechtszuordnung des Vogels getroffen werden kann.

Durch die Druckschrift DE 10 2010 006 161 B3 ist eine Verfahren und eine Vorrichtung zur Bestimmung des Geschlechts von befruchteten und nicht bebrüteten Vogeleiern bekannt, wobei ein Ei eine feste Eischale, ein von der Eischale und weiteren Eihüllen umgebenes Eidotter und eine dem Eidotter zugeordnete Keimscheibe aufweist. Dabei wird eine Sonde zur Messung eines Spektrums durch ein Loch in der Eischale hindurch in Richtung zur Keimscheibe mit Keimscheibenzellen geführt. Mit der Sonde werden die Keimscheibenzellen spektroskopisch untersucht und das Spektrum klassifiziert. Zur Bestimmung des Geschlechts ist ein nicht unerheblicher Aufwand notwendig, wobei die Eischale gelocht werden muss.

Die Druckschrift EP 2 336 751 B1 zeigt ein Verfahren zur Bestimmung des Geschlechts an Vogeleiern, wobei mit einer Strahlungsquelle elektromagnetische Strahlung auf die Keimscheibe eines unbebrüteten Eies emittiert und nach dem Abschalten der Strahlungsquelle am bestrahlten Bereich der Keimscheibe das Abklingverhalten der durch die elektromagnetische Strahlung angeregten Eigenfluoreszenzintensität zeit- und spektralaufgelöst für mindestens eine Wellenlänge der Eigenfluoreszenz mit einem Detektor erfasst wird. Daraus wird das jeweilige Ei als weiblich oder männlich eingestuft.

Die Druckschrift DE 10 2019 009 002 A1 betrifft eine Einrichtung zur Untersuchung von Vogeleiern, wobei wenigstens ein Träger mit beabstandet zueinander angeordneten Vogeleiern zwischen wenigstens einer Quelle für elektromagnetische Wellen und einer Farbkamera und/oder einer Hyperspektralkamera angeordnet ist. Der Träger besitzt Ausschnitte zur Platzierung der Vogeleier. Zwischen den Vogeleiern befinden sich Trennwände. Weiterhin sind die Quelle für elektromagnetische Wellen und die Farbkamera und/oder die Hyperspektralkamera mit einem Datenverarbeitungssystem verbunden, welches aus dem Abbild der Farbkamera und/oder den von der Hyperspektralkamera aufgenommenen Transmissionsspektren Merkmale ermittelt und einem Embryo und damit dem jeweiligen Vogelei zuordnet. Bei einer derartigen Anordnung ist insbesonder Streulicht, welches die Untersuchung und damit die Bestimmung des jeweiligen Vogeleies beeinflusst, und daraus resultierende fehlerhafte Bestimmungen der Embryonen nicht auszuschließen. Weiterhin sind im früheren Stadium weniger ausgebildete geschlechtsspezifische Merkmale der Embryonen nicht zweifelsfrei ermittelbar.

Die Druckschrift DE 10 2018 001 987 A1 betrifft eine Einrichtung zur Untersuchung von Bruteiern ab dem sechzehnten Bebrütungstag. Dazu ist ein Träger mit beabstandet zueinander angeordneten Bruteiern zwischen einer Quelle für elektromagnetische Wellen und einer Hyperspektralkamera angeordnet. Der Träger besitzt weiterhin Ausschnitte zur Platzierung der Bruteier und lichtdichten Ankopplung der elektromagnetischen Wellen besitzt. Zwischen den Bruteiern befinden sich Trennwände. Die Quelle für elektromagnetische Wellen und die Hyperspektralkamera sind mit einem Datenverarbeitungssystem verbunden, welches aus dem Abbild der Hyperspektralkamera die Eiabbilder ermittelt und dem jeweiligen Eiabbild hyperspektrale Daten und die Position des jeweiligen Bruteies auf dem Träger zuordnet.

Die Druckschrift DE 600 03 914 T2 beinhaltet ein Verfahren und eine Vorrichtung zur selektiven Klassifizierung von Hühnereiern, wobei klare Eier aus einer Vielzahl von Eiern unter Verwendung der Eieropatitäten identifiziert werden. Es wird ein räumlicher Temperaturtrend aus der Vielzahl der Eier unter Verwendung der Identifikation der klaren Eier bestimmt. Daraus werden lebende Eier aus der Vielzahl der Eier unter Verwendung des räumlichen Temperaturtrends identifiziert. Zur Ermittlung der Eieropatitäten weist die Einrichtung ein Licht-Durchleuchtungssystem auf. Weiterhin besitzt die Einrichtung zur Erfassung der Eiertemperaturen ein thermisches Durchleuchtungssystem, welches die Eiertemperaturen erfasst und Temperatursignale erzeugt, die den Eiertemperaturen entsprechen. Dazu werden als Licht-Durchleuchtungssystem ein Infarotstrahler und ein Infrarotdetektor und als thermisches Durchleuchtungssystem ein Infrarotsensor verwendet.

Die Druckschrift US 2 987 182 A betrifft eine Einrichtung zur Untersuchung auf das Vorhandensein oder das Fehlen von Blut in Eiern. Dazu besitzt die Einrichtung einer Förderer für Eier und eine Rinne zur Platzierung und Untersuchung eines Eies mittels einer Lichtquelle und photoelektrischen Detektoren.

Durch die Druckschrift CN 1 742 569 A ist ein Verfahren und eine Vorrichtung zur Bestimmung des Geschlechts von Haushühnern in der frühen Inkubationsphase von Embryoeiern bekannt. Die Vorrichtung weist insbesondere eine Digitalkamera und eine Lichtquelle auf. Grundlage bildet das Erstellen eines Blutliniendiagramms eines bebrüteten embryonalen Eies.

Der im Patentanspruch 1 angegebenen Erfindung liegt die Aufgabe zugrunde, ein Vorhandensein von Embryonen in Bruteiern und deren Geschlecht zu ermitteln.

Diese Aufgabe wird mit den im Patentanspruch 1 aufgeführten Merkmalen gelöst.

Die Einrichtung zur Untersuchung von Bruteiern mit einer Transporteinrichtung, Lichtquellen und Kameras in Verbindung mit einem Datenverarbeitungssystem zeichnet sich insbesondere dadurch aus, dass ein Vorhandensein von Embryonen in Bruteiern und deren Geschlecht ermittelbar ist.

Dazu besitzt die Transporteinrichtung aneinander angeordnete Halter zur Aufnahme von Bruteiern. Die Halter befinden sich auf zwei beabstandet parallel zueinander angeordneten Riementrieben. Die Halter weisen Grundplatten mit in einer Reihe beabstandet zueinander angeordneter Öffnungen zur Platzierung von Bruteiern, u-förmig angeordnete Wände und Rohrstücke auf, so dass sich die Öffnungen und die Rohrstücke der Halter zwischen den Riementrieben befinden, die Wände benachbart angeordneter Halter rohrförmig ausgebildet sind und die Rohrstücke und die Wände erste Lichtschächte zur Platzierung von Bruteiern und bei Platzierung mit Bruteiern sind. Unter Haltern befinden sich in einer Matrix oder in Matrizen angeordnete Lichtquellen, wobei die Lichtquellen zur Beaufschlagung von Bruteiern mehrerer Halter mit elektromagnetischen Wellen beabstandet zu Bruteiern in zweiten Lichtschächten angeordnet sind. Die Kameras sind über Haltern angeordnet, so dass zur Untersuchung von Bruteiern die ersten Lichtschächte und die zweiten Lichtschächte gemeinsame Lichtschächte ausbilden und sich Bruteier von Reihen oder von Bereichen von Reihen zwischen Lichtquellen und Kameras befinden. Die Kameras sind mit dem Datenverarbeitungssystem zur Erkennung von Leerstellen und der Zustände von Bruteiern verbunden.

Die Halter bilden nacheinander angereiht vorteilhafterweise die ersten Lichtschächte zur Platzierung jeweils eines Bruteies in einem Lichtschacht und damit erste Lichtschächte mit jeweils einem Brutei. Die mit den Öffnungen der Grundplatten verbundenen Rohrstücke sind erste Bestandteile der ersten Lichtschächte. Die Schenkel der u-förmigen Wände sind seitliche Wände auf den Grundplatten oder den Rohrstücken und stellen die zweiten Bestandteile der ersten Lichtschächte dar. Weiterhin sind die Mittelteile der u-förmigen Wände eines Halters jeweils sowohl Rückwände der zweiten Bestandteile erster Lichtschächte als auch Vorderwände der zweiten Bestandteile erster Lichtschächte des nachgeordneten Halters. Bei Positionierung erster Lichtschächte mit Bruteiern korrespondierend zu den zweiten Lichtschächten mit den Lichtquellen sind durchgängige Lichtschächte mit jeweils einem Brutei und einer Lichtquelle ausgebildet. Damit ist jedem zu untersuchenden Brutei eine Lichtquelle zuordenbar. Gleichzeitig wird Streulicht bei der Untersuchung von Bruteiern weitestgehend vermieden, so dass eine verbesserte Untersuchung und damit sichere Bestimmung des jeweiligen Bruteies mittels den Kameras gegeben ist.

Die Querschnitte der Öffnungen und der Rohrstücke sind insbesondere Kreise. Der Durchmesser der Öffnungen kann dazu kleiner oder gleich der Durchmesser der Rohrstücke sein. Die Öffnungen und/oder Rohrstücke sind weiterhin vorteilhafterweise gleichzeitig Halterungen der Bruteier. Dazu sind die Durchmesser der Öffnungen und/oder der Rohrstücke kleiner als die größten Querschnitte der Bruteier. Die Länge der Rohrstücke ist dabei so groß, dass platzierte Bruteier die Halter nicht überragen.

Die Endenbereiche der Schenkel der u-förmigen Wände der Halter können weiterhin abgewinkelt sein. Gleichzeitig können die Mittelteile der u-förmigen Wände in Richtung nachfolgender Halter Wandabschnitte besitzen, welche bereichsweise die abgewinkelten Endenbereiche der Schenkel der u-förmigen Wände des nachfolgenden Halters überlappen. Damit werden Spalte zwischen ersten Lichtschächten der nacheinander angeordneten Halter während der Untersuchung der Bruteier weitestgehend verringert.

Die Halter befinden sich dazu auf parallel beabstandet zueinander angeordneten Riementrieben, so dass ein durchgängiges Transportband mit den Haltern ausgebildet ist. Gleichzeitig sind platzsparende Umlenkungen vorhanden. Die zweiten Lichtschächte mit den Lichtquellen befinden sich zwischen den Umlenkungen und dem durchgängigen Transportband.

Vorteilhafte Ausgestaltungen der Erfindung sind in den folgenden Weiterbildungen und Ausführungsformen aufgeführt. Diese können die Einrichtung zur Untersuchung von Bruteiern mit einer Transporteinrichtung, Lichtquellen und Kameras in Verbindung mit einem Datenverarbeitungssystem einzeln oder in einer Kombination fortbilden.

Zwischen korrespondierend zueinander angeordneten Lichtschächten befindet sich in einer Ausführungsform mindestens eine thermisch stabile Glasscheibe oder Glaskeramikscheibe mit an die Lichtquellen angepasster optischer Transmittivität. Damit ist insbesondere ein Schutz der Lichtquellen vor beschädigten Bruteiern gegeben. Gleichzeitig sind die einzelnen Halter und die Glasscheibe leicht reinigbar.

Die in einer Matrix angeordneten Lichtquellen sind in einer Ausführungsform eine Baugruppe mit Vertiefungen zur Aufnahme der Lichtquellen. Weiterhin besitzt die Baugruppe wenigstens eine Kühleinrichtung. Das kann ein passiver und/oder ein aktiver Kühlkörper sein. Darüber hinaus kann die Baugruppe wenigstens einen Kühlkanal mit Anschlüssen für ein fließfähiges Medium aufweisen. Die Baugruppe kann dabei auch aus miteinander verbundenen Bauteilen bestehen, so dass ein durchgängiger Kühlkanal für die Vertiefungen mit den Lichtquellen leicht realisierbar.

In Transportrichtung der Bruteier sind in einer Ausführungsform unter Haltern wenigstens eine erste und eine zweite Baugruppe mit Lichtquellen und wenigstens eine erste und wenigstens eine zweite Kamera angeordnet. Die erste Baugruppe und die erste Kamera dienen der Ermittlung von Leerstellen und Bruteiern mit erhöhter oder vermindeter Intensität/Transmittivität. Letztere tritt insbesondere bei stark verschmutzten, keimbelasteten oder zu weit entwickelten Bruteiern auf. Die zweite Baugruppe und die zweite Kamera wird vorteilhafterweise zur Ermittlung unterschiedlicher optischer Eigenschaften der Geschlechter der Embryonen verwendet.

Dazu können die Kameras beispielsweise Farbkameras, Hyperspektral-Flächenkameras und/oder Hyperspektral-Zeilenkameras sein. Günstigerweise kann für die erste Gruppe eine Farbkamera und für die zweite Gruppe wenigstens eine Hyperspektralkamera verwendet werden. Dabei können auch Gruppen von Zeilenkameras eingesetzt werden.

Das Datenverarbeitungssystem ist in einer Ausführungsform so ausgebildet, dass während der Untersuchung
- eine erhöhte Intensität/Transmittivität,
- eine herabgesetzte Intensität/Transmittivität,
- ein Vorhandensein wenigstens eines Pigmentfarbstoffes bei einem weiblichen Embryo oder
- ein Vorhandensein wenigstens eines geschlechtsspezifischen optischen Merkmals bei wenigstens einem der beiden Geschlechter
bestimmt wird und der jeweiligen Position des Bruteies in dem Halter zugeordnet wird.

Eine erhöhte Intensität/Transmittivität tritt insbesondere bei einem unbefruchteten Ei, unterentwickelten Embryonen oder Leerstellen auf. Eine herabgesetzte Intensität/Transmittivität kann insbesondere einem keimbelasteten Brutei, einem zu weit entwickelten Brutei oder einem verschmutzten Brutei zugeordnet werden. Diese Bruteier werden entfernt und nicht weiter bebrütet.

Die Lichtquellen sind in einer Ausführungsform mit dem Datenverarbeitungssystem so zusammengeschaltet, dass die Lichtquellen entsprechend der Bestimmungen der Bruteier einschaltbar, ausschaltbar und in ihren Lichtleistungen anpassbar sind. Letzteres erfolgt insbesondere durch Dimmen. Dabei können als Lichtquellen wenigstens der zweiten Baugruppe Lichtquellen mit einer Wellenlänge jeweils einschließlich von 400 nm bis 1000 nm verwendet werden. Das können insbesondere Halogenglühlampen oder Leuchtdioden sein.

Auf der thermisch stabilen Glasscheibe oder Glaskeramikscheibe befindet sich in einer Ausführungsform eine Blende mit kreisrunden korrespondierend zu den Lichtquellen angeordneten Ausschnitten.

In einer Ausführungsform ist oder sind der Einrichtung zur Untersuchung von Bruteiern eine zu untersuchende Bruteier den Haltern zuführende Vorrichtung und/oder wenigstens eine Bruteier von Haltern abführende Vorrichtung zugeordnet. Die Bruteier zuführende Vorrichtung und/oder die Bruteier abführende Vorrichtung weist oder weisen jeweils einen Träger mit in Matrizen angeordneten Halteelementen auf. Weiterhin ist der Träger mit einem Linearantrieb und einer Hubvorrichtung verbunden.

Halteelemente des Trägers sind in einer Ausführungsform zusammen angetrieben und geführt so verschiebbar, dass sich der Abstand der Halteelemente zueinander vergrößert oder verkleinert. In Fortführung können die Führungen einer Reihe quer zur Transportrichtung der Bruteier geradlinig und fächerförmig verlaufend ausgebildet sein.

Ein Ausführungsbeispiel der Erfindung ist in den Zeichnungen jeweils prinzipiell dargestellt und wird im Folgenden näher beschrieben.

Es zeigen:
- Fig. 1: eine Einrichtung zur Untersuchung von Bruteiern,
- Fig. 2: einen Ausschnitt eines Halters in Verbindung mit einer Lichtquelle zur Untersuchung eines Bruteies in einer Seitenansicht und
- Fig. 3: einen Ausschnitt eines Halters in einer Draufsicht.

Eine Einrichtung zur Untersuchung von Bruteiern besteht im Wesentlichen aus einer Transporteinrichtung 1, Lichtquellen 2 und Kameras 3 in Verbindung mit einem Datenverarbeitungssystem 4.

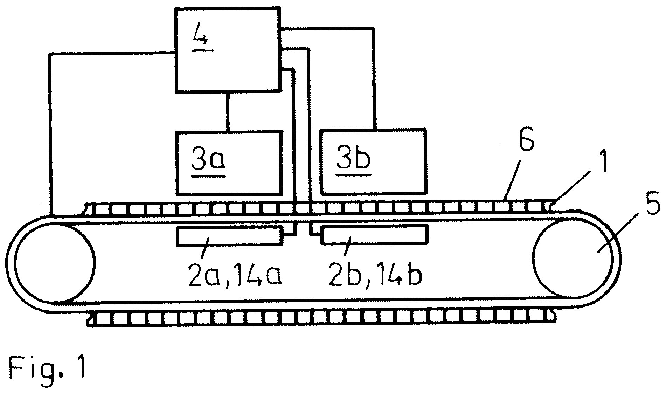

Die Fig. 1 zeigt eine Einrichtung zur Untersuchung von Bruteiern in einer prinzipiellen Darstellung.

Die Transporteinrichtung 1 besitzt aneinander angeordnete Halter 6 zur Aufnahme von Bruteiern 7. Die Halter 6 befinden sich auf zwei beabstandet parallel zueinander angeordneten Riementrieben 5 und bilden so ein durchgängiges Transportband aus. Wenigstens einer der Riementriebe 5 ist mit einem Antrieb verbunden, welcher weiterhin mit dem Datenverarbeitungssystem 4 verbunden ist. Über der Transporteinrichtung 1 sind die Kameras 3 angeordnet. Dazu sind wenigstens eine erste Kamera 3a und wenigstens eine zweite Kamera 3a in Transportrichtung der Bruteier 7 nacheinander angeordnet. Die Lichtquellen 2 befinden sich zwischen dem durch die Riementriebe 5 in Verbindung mit den Haltern 6 ausgebildeten durchgängigen Transportband sowie deren Umlenkungen. Dabei sind erste Lichtquellen 2a und zweite Lichtquellen 2b in Transportrichtung der Bruteier 7 nacheinander angeordnet.

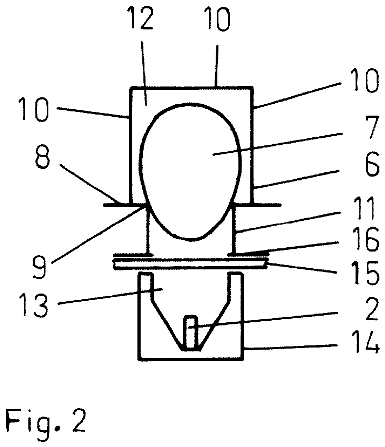

Die Fig. 2 zeigt einen Ausschnitt eines Halters 6 in Verbindung mit einer Lichtquelle 2 zur Untersuchung eines Bruteies 7 in einer prinzipiellen Seitenansicht.

Die Halter 6 besitzen Grundplatten 8 mit in einer Reihe beabstandet zueinander angeordneter Öffnungen 9 zur Platzierung von Bruteiern 7, u-förmig angeordnete Wände 10 und Rohrstücke 11. Die Öffnungen 9 und die Rohrstücke 11 der Halter 6 befinden sich zwischen den Riementrieben 5. Die Wände 10 benachbart angeordneter Halter 6 sind rohrförmig ausgebildet. Die Wände 10 und die Rohrstücke 11 sind erste Lichtschächte 12 zur Platzierung von Bruteiern 7 und bei Platzierung mit Bruteiern 7. Unter den Haltern 6 befinden sich die in einer Matrix oder in Matrizen angeordneten Lichtquellen 2a, 2b, wobei die Lichtquellen 2a, 2b zur Beaufschlagung von Bruteiern 7 mehrerer Halter 6 mit elektromagnetischen Wellen beabstandet zu Bruteiern 7 in zweiten Lichtschächten 13 angeordnet sind. Die in einer Matrix angeordneten Lichtquellen 2a, 2b sind jeweils eine Baugruppe 14 mit Vertiefungen 13 zur Aufnahme der Lichtquellen 2a, 2b. Die Vertiefungen 13 sind gleichzeitig die zweiten Lichtschächte 13. Die Baugruppe 14 kann wenigstens einen Kühlkanal mit Anschlüssen für ein fließfähiges Medium besitzen. Zwischen korrespondierend zueinander angeordneten Lichtschächten 12, 13 kann sich mindestens eine thermisch stabile Glasscheibe 15 oder Glaskeramikscheibe mit an die Lichtquellen 2 angepasster optischer Transmittivität befinden. Weiterhin kann sich auf der thermisch stabilen Glasscheibe 15 oder Glaskeramikscheibe eine Blende 16 mit kreisrunden korrespondierend zu den Lichtquellen 2 angeordneten Ausschnitten befinden.

Die Kameras 3a, 3b sind über Haltern 6 angeordnet, so dass zur Untersuchung von Bruteiern 7 die ersten Lichtschächte 12 und die zweiten Lichtschächte 13 gemeinsame Lichtschächte ausbilden und sich Bruteier 7 von Reihen oder von Bereichen von Reihen zwischen Lichtquellen 2 und Kameras 3a, 3b befinden. Die Kameras 3a, 3b sind mit dem Datenverarbeitungssystem 4 zur Erkennung von Leerstellen und der Zustände von Bruteiern 7 verbunden.

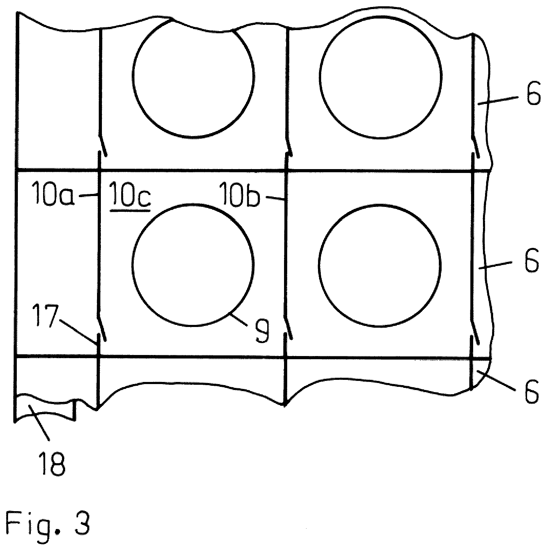

Die Fig. 3 zeigt einen Ausschnitt eines Halters 6 in einer prinzipiellen Draufsicht.

Die Halter 6 besitzen die u-förmig angeordneten Wände 10. Die Endenbereiche der Schenkel 10a, 10b der u-förmigen Wände 10 der Halter 6 können abgewinkelt sein. Gleichzeitig können die Mittelteile 10c der u-förmigen Wände 10 in Richtung nachfolgender Halter 6 Wandabschnitte 17 besitzen, welche bereichsweise die abgewinkelten Endenbereiche der Schenkel 10a, 10b der u-förmigen Wände 10 des nachfolgenden Halters 6 überlappen. Damit werden Spalte zwischen ersten Lichtschächten 12 der nacheinander angeordneten Halter 6 während der Untersuchung der Bruteier 7 weitestgehend verringert. Die Halter 6 sind auf den Riementrieben 5 befestigt. Als Riemen 18 können dazu Zahnriemen 18 verwendet werden.

In Transportrichtung der Bruteier 7 sind unter Haltern 6 wenigstens eine erste Baugruppe 14a mit Lichtquellen 2a und eine zweite Baugruppe 14b mit Lichtquellen 2b und wenigstens eine erste Kamera 3a und wenigstens eine zweite Kamera 3b angeordnet. Die erste Baugruppe 14a und die erste Kamera 3a dienen der Ermittlung von Leerstellen und Bruteiern mit erhöhter Intensität/Transmittivität. Die zweite Baugruppe 14b und die zweite Kamera 3b dienen der Ermittlung der Geschlechter der Embryonen. Die Kameras 3a, 3b können dazu Flächenkameras 3a, 3b und/oder Zeilenkameras 3a, 3b und/oder eine Flächenkamera 3a und wenigstens eine Zeilenkamera 3b sein. Dazu können Farbkameras 3a, 3b und/oder Hyperspektralkameras 3a, 3b verwendet werden.

Die Lichtquellen 2 können vorteilhafterweise Wellenlängen jeweils einschließlich von 400 nm bis 1000 nm besitzen. Das können Halogenglühlampen oder Leuchtdiodensein.

Das Datenverarbeitungssystem 4 ist so ausgebildet, dass während der Untersuchung
- eine erhöhte Intensität/Transmittivität, beispielsweise bei einem unbefruchteten Ei 7, unterentwickelten Embryonen oder Leerstellen,
- eine herabgesetzte Intensität/Transmittivität, beispielsweise bei einem keimbelastetem Brutei 7, zu weit entwickeltem Brutei 7, verschmutztem Brutei 7,
- ein Vorhandensein wenigstens eines Pigmentfarbstoffes bei einem weiblichen Embryo
   oder
- ein Vorhandensein wenigstens eines geschlechtsspezifischen Merkmals bei einem weiblichen Embryo
bestimmt wird und der jeweiligen Position des Bruteies 7 in dem Halter 6 zugeordnet wird.

Die Lichtquellen 2 können mit dem Datenverarbeitungssystem 4 so zusammengeschaltet sein, dass die Lichtquellen 2 entsprechend der Bestimmungen der Bruteier 7 einschaltbar, ausschaltbar und in ihren Lichtleistungen anpassbar sind. So sind Lichtquellen 2 dimmbar.

In einer Ausführungsform kann oder können der Einrichtung zur Untersuchung von Bruteiern 7 eine zu untersuchende Bruteier 7 den Haltern 6 zuführende Vorrichtung und/oder wenigstens eine Bruteier 7 von Haltern 6 abführende Vorrichtung zugeordnet sein. Dazu kann oder können die Bruteier 7 zuführende Vorrichtung und/oder die Bruteier 7 abführende Vorrichtung jeweils einen Träger mit in Matrizen angeordneten Halteelementen aufweisen. Der Träger kann weiterhin mit einem Linearantrieb und einer Hubvorrichtung verbunden sein. Halteelemente können dazu beispielsweise Saugnäpfe sein. Die Halteelemente des Trägers können zusammen angetrieben und geführt so verschiebbar sein, dass sich der Abstand der Halteelemente zueinander vergrößert oder verkleinert. Dazu können die Führungen einer Reihe quer zur Transportrichtung der Bruteier geradlinig und fächerförmig verlaufend ausgebildet sein.

## Patentansprüche

1. Einrichtung zur Untersuchung von Bruteiern (7) mit einer Transporteinrichtung (1), Lichtquellen (2) und Kameras (3) in Verbindung mit einem Datenverarbeitungssystem (4), **dadurch gekennzeichnet, dass** die Transporteinrichtung (1) aneinander angeordnete Halter (6) zur Aufnahme von Bruteiern (7) besitzt, dass sich die Halter (6) auf zwei beabstandet parallel zueinander angeordneten Riementrieben (5) befinden, dass die Halter (6) Grundplatten (8) mit in einer Reihe beabstandet zueinander angeordneter Öffnungen (9) zur Platzierung von Bruteiern (7), u-förmig angeordnete Wände (10) und Rohrstücke 11 aufweisen, so dass sich die Öffnungen (9) und die Rohrstücke (11) der Halter (6) zwischen den Riementrieben (5) befinden, die Wände (10) benachbart angeordneter Halter (6) rohrförmig ausgebildet sind und die Wände (10) und die Rohrstücke (11) erste Lichtschächte (12) zur Platzierung von Bruteiern (7) und bei Platzierung mit Bruteiern (7) sind, dass sich in einer Matrix oder in Matrizen angeordnete Lichtquellen (2) unter Haltern (6) befinden, wobei die Lichtquellen (2) zur Beaufschlagung von Bruteiern (7) mehrerer Halter (6) mit elektromagnetischen Wellen beabstandet zu Bruteiern (7) in zweiten Lichtschächten (13) angeordnet sind, dass die Kameras (3) über Haltern (6) angeordnet sind, so dass zur Untersuchung von Bruteiern (7) die ersten Lichtschächte (12) und die zweiten Lichtschächte (13) gemeinsame Lichtschächte ausbilden und sich Bruteier (7) von Reihen oder von Bereichen von Reihen zwischen Lichtquellen (2) und Kameras (3) befinden und dass die Kameras (3) mit dem Datenverarbeitungssystem (4) zur Erkennung von Leerstellen und der Zustände von Bruteiern (7) verbunden sind.

2. Einrichtung nach Patentanspruch 1, **dadurch gekennzeichnet, dass** sich zwischen korrespondierend zueinander angeordneten Lichtschächten mindestens eine thermisch stabile Glasscheibe (15) oder Glaskeramikscheibe mit an die Lichtquellen (2) angepasster optischer Transmittivität befindet.

3. Einrichtung nach wenigstens einem der Patentansprüche 1 und 2, **dadurch gekennzeichnet, dass** die in einer Matrix angeordneten Lichtquellen (2) eine Baugruppe (14) mit Vertiefungen zur Aufnahme der Lichtquellen (2) sind und dass die Baugruppe (14) wenigstens eine Kühleinrichtung besitzt.

4. Einrichtung nach wenigstens einem der Patentansprüche 1 bis 3, **dadurch gekennzeichnet, dass** in Transportrichtung der Bruteier (7) unter Haltern 6 wenigstens eine erste Baugruppe (14a) und eine zweite Baugruppe (14b) mit Lichtquellen (2) und wenigstens eine erste Kamera (3a) und wenigstens eine zweite Kamera (3b) angeordnet sind, wobei die erste Baugruppe (14a) und die erste Kamera (3a) der Ermittlung von Leerstellen und Bruteiern (7) mit erhöhter oder vermindeter Intensität/Transmittivität und wobei die zweite Baugruppe (14b) und die zweite Kamera (3b) der Ermittlung unterschiedlicher optischer Eigenschaften der Geschlechter der Embryonen dienen.

5. Einrichtung nach wenigstens einem der Patentansprüche 1 bis 4, **dadurch gekennzeichnet, dass** das Datenverarbeitungssystem (4) so ausgebildet ist, dass während der Untersuchung
- eine erhöhte Intensität/Transmittivität,
- eine herabgesetzte Intensität/Transmittivität,
- ein Vorhandensein wenigstens eines Pigmentfarbstoffes bei einem weiblichen Embryo
oder
- ein Vorhandensein wenigstens eines geschlechtsspezifischen optischen Merkmals bei wenigstens einem der beiden Geschlechter
bestimmt wird und der jeweiligen Position des Bruteies (7) in dem Halter (6) zugeordnet wird.

6. Einrichtung nach wenigstens einem der Patentansprüche 1 bis 5, **dadurch gekennzeichnet, dass** die Lichtquellen (2) mit dem Datenverarbeitungssystem (4) so zusammengeschaltet sind, dass die Lichtquellen (2) entsprechend der Bestimmungen der Bruteier (7) einschaltbar, ausschaltbar und in ihren Lichtleistungen anpassbar sind.

7. Einrichtung nach wenigstens einem der Patentansprüche 1 bis 6, **dadurch gekennzeichnet, dass** sich auf der thermisch stabilen Glasscheibe (15) oder Glaskeramikscheibe eine Blende (16) mit kreisrunden korrespondierend zu den Lichtquellen (2) angeordneten Ausschnitten befindet.

8. Einrichtung nach wenigstens einem der Patentansprüche 1 bis 7, **dadurch gekennzeichnet, dass** der Einrichtung zur Untersuchung von Bruteiern (7) eine zu untersuchende Bruteier (7) den Haltern (6) zuführende Vorrichtung und/oder wenigstens eine Bruteier (7) von Haltern (6) abführende Vorrichtung zugeordnet ist oder sind, dass die Bruteier (7) zuführende Vorrichtung und/oder die Bruteier (7) abführende Vorrichtung jeweils einen Träger mit in Matrizen angeordneten Halteelementen aufweist oder aufweisen und dass der Träger mit einem Linearantrieb und einer Hubvorrichtung verbunden ist.

9. Einrichtung nach wenigstens einem der Patentansprüche 1 bis 8, **dadurch gekennzeichnet, dass** Halteelemente des Trägers zusammen angetrieben und geführt so verschiebbar sind, dass sich der Abstand der Halteelemente zueinander vergrößert oder verkleinert.

10. Einrichtung nach wenigstens einem der Patentansprüche 1 bis 9, **dadurch gekennzeichnet, dass** die Führungen einer Reihe quer zur Transportrichtung der Bruteier (7) geradlinig und fächerförmig verlaufend ausgebildet sind.

## Claims

1. A device for examining hatching eggs (7) with a transport device (1), light sources (2) and cameras (3) in connection with a data processing system (4), **characterized in that** the transport device (1) has holders (6) arranged next to one another for receiving hatching eggs (7), that the holders (6) are located on two belt drives (5) arranged at a distance parallel to one another, that the holders (6) have base plates (8) with openings (9) arranged in a row at a distance from one another for placing hatching eggs (7), walls (10) arranged in a U-shape and tubular pieces 11 so that the openings (9) and the tubular pieces (11) of the holders (6) are located between the belt drives (5), the walls (10) of adjacently arranged holders (6) are tubular, and the walls (10) and the tubular pieces (11) are first light shafts (12) for placing hatching eggs (7) and when placing with hatching eggs (7), that light sources (2) arranged in a matrix or in matrices are located under holders (6), wherein the light sources (2) are arranged at a distance from hatching eggs (7) in second light shafts (13) for exposing hatching eggs (7) in several holders (6) to electromagnetic waves, that the cameras (3) are arranged above holders (6) so that the first light shafts (12) and the second light shafts (13) form common light shafts for examining hatching eggs (7), and hatching eggs (7) from rows or from regions of rows are located between light sources (2) and cameras (3), and that the cameras (3) are connected to the data processing system (4) for detecting empty spaces and the states of hatching eggs (7).

2. The device according to claim 1, **characterized in that** between light shafts arranged corresponding to one another, there is at least one thermally stable glass pane (15) or glass ceramic pane with optical transmittivity adapted to the light sources (2).

3. The device according to at least one of claims 1 and 2, **characterized in that** the light sources (2) arranged in a matrix are an assembly (14) with recesses for receiving the light sources (2), and that the assembly (14) possesses at least one cooling device.

4. The device according to at least one of claims 1 to 3, **characterized in that** in the transport direction of the hatching eggs (7), at least one first assembly (14a) and one second assembly (14b) with light sources (2) and at least one first camera (3a) and at least one second camera (3b) are arranged under holders 6, wherein the first assembly (14a) and the first camera (3a) serve to determine empty spaces and hatching eggs (7) with increased or reduced intensity/transmittivity, and wherein the second assembly (14b) and the second camera (3b) serve to determine different optical properties of the sexes of the embryos.

5. The device according to at least one of claims 1 to 4, **characterized in that** the data processing system (4) is designed such that during the examination:
- increased intensity/transmittivity,
- reduced intensity/transmittivity,
- a presence of at least one pigment in a female embryo, or
- a presence of at least one sex-specific optical characteristic of at least one of the two sexes
is determined and assigned to the respective position of the hatching egg (7) in the holder (6).

6. The device according to at least one of claims 1 to 5, **characterized in that** the light sources (2) are connected to the data processing system (4) in such a way that the light sources (2) can be switched on, switched off, and their light outputs can be adjusted according to the determination of the hatching eggs (7).

7. The device according to at least one of claims 1 to 6, **characterized in that** a diaphragm (16) with circular cutouts arranged corresponding to the light sources (2) is located on the thermally stable glass pane (15) or glass ceramic pane.

8. The device according to at least one of claims 1 to 7, **characterized in that** the device for examining hatching eggs (7) is assigned a device supplying hatching eggs (7) to be examined to the holders (6) and/or at least one device for removing hatching eggs (7) from holders (6), that the device supplying the hatching eggs (7) and/or the device removing the hatching eggs (7) each has or have a carrier with holding elements arranged in matrices, and that the carrier is connected to a linear drive and a lifting device.

9. The device according to at least one of claims 1 to 8, **characterized in that** holding elements of the carrier are driven together and moveably guided so that the distance of the holding elements to each other increases or decreases.

10. The device according to at least one of claims 1 to 9, **characterized in that** the guides of a row are designed to run straight and fan-shaped transversely to the transport direction of the hatching eggs (7).

## Revendications

1. Dispositif d'examen d'œufs à couver (7) avec un dispositif de transport (1), des sources lumineuses (2) et des caméras (3) en liaison avec un système de traitement de données (4), **caractérisé en ce que** le dispositif de transport (1) comporte des supports (6) disposés côte à côte pour recevoir les œufs à couver (7), **en ce que** les supports (6) sont situés sur deux entraînements par courroie (5) parallèles, **en ce que** les supports (6) comportent des plaques de base (8) munies d'ouvertures (9) espacées les unes des autres pour le placement des œufs à couver (7), de parois en U (10) et de sections de tube (11), de manière que les ouvertures (9) et les sections de tube (11) des supports (6) sont situés entre les entraînements par courroie (5), **en ce que** les parois (10) des supports (6) adjacents sont tubulaires et constituent des puits de lumière (12) pour le placement des œufs à couver (7) lors du placement avec des œufs à couver (7), **en ce que** des sources lumineuses (2) disposées dans une matrice ou plusieurs matrices se trouvent sous des supports (6), les sources lumineuses (2) destinées à irradier les œufs à couver (7) de plusieurs supports (6) avec des ondes électromagnétiques etant disposées à distance des œufs (7) dans des seconds puits de lumière (13), **en ce que** les caméras (3) sont disposées au-dessus des supports (6) de manière que, pour l'examen des œufs (7), les premiers puits de lumière (12) et les seconds puits de lumière (13) forment des puits de lumière communs et des œufs à couver (7) des rangées ou des zones de rangées sont situés entre les sources lumineuses (2) et les caméras (3) Et **en ce que** les caméras (3) sont liées au système de traitement de données (4) pour la détection des espaces vides et l'état des œufs à couver (7).

2. Dispositif selon la revendication 1, **caractérisé en ce qu'**au moins une vitre en verre (15) ou en vitrocéramique thermostable, dont la transmittance optique est adaptée aux sources lumineuses (2), est située entre les puits de lumière disposés de manière correspondante.

3. Dispositif selon au moins l'une des revendications 1 et 2, **caractérisé en ce que** les sources lumineuses (2) disposées en matrice constituent un ensemble (14) doté d'évidements pour l'accueil des sources lumineuses (2), et **en ce que** l'ensemble (14) comporte au moins un dispositif de refroidissement.

4. Dispositif selon au moins l'une des revendications 1 à 3, **caractérisé en ce qu'**au moins un premier ensemble (14a) et un second ensemble (14b) comprenant des sources lumineuses (2) et au moins une première caméra (3a) et au moins une seconde caméra (3b) sont disposés sous les supports 6 dans le sens de transport des œufs à couver (7), le premier ensemble (14a) et la première caméra (3a) servant à la détection des espaces vides et les œufs à couver (7) présentant une intensité/transmittance accrue ou réduite, et le second ensemble (14b) et la seconde caméra (3b) servant à la détection les différentes propriétés optiques des sexes des embryons.

5. Dispositif selon au moins l'une des revendications 1 à 4, **caractérisé en ce que** le système de traitement de données (4) est configuré de telle maniére que, lors de l'examen,
- une intensité/transmittance accrue,
- une intensité/transmittance diminuée,
- la présence d'au moins un colorant pigmentaire chez un embryon femelle, ou
- la présence d'au moins une caractéristique optique spécifique au sexe chez au moins l'un des deux sexes
est déterminée et attribuée à la position respective de l'œuf à couver (7) dans le support (6).

6. Dispositif selon au moins l'une des revendications 1 à 5, **caractérisé en ce que** les sources lumineuses (2) sont interconnectées au système de traitement de données (4) de telle manière que les sources lumineuses (2) peuvent être allumées, éteintes et leur puissance lumineuse peut être réglée en fonction des déterminations des œufs à couver (7).

7. Dispositif selon au moins l'une des revendications 1 à 6, **caractérisé en ce qu'**un diaphragme (16) à découpes circulaires disposées en fonction des sources lumineuses (2) est situé sur la vitre en verre (15) ou en vitrocéramique thermostable.

8. Dispositif selon au moins l'une des revendications 1 à 7, **caractérisé en ce qu'**un dispositif d'alimentation des œufs à couver (7) à examiner vers les supports (6) et/ou à au moins un dispositif de prélèvement des œufs à couver (7) des supports (6) est ou sont associés au dispositif d'examen des œufs à couver (7), **en ce que** le dispositif d'alimentation des œufs à couver (7) et/ou le dispositif de prélèvement des œufs à couver (7) comportent chacun un porteur avec des éléments de support disposés en matrices, et **en ce que** le porteur est relié à un entraînement linéaire et à un dispositif de levage.

9. Dispositif selon au moins l'une des revendications 1 à 8, **caractérisé en ce que** des éléments de support du porteur sont entraînés et guidés ensemble sont déplaçable de sorte que la distance entre eux augmente ou diminue.

10. Dispositif selon au moins l'une des revendications 1 à 9, **caractérisé en ce que** les guides d'une rangée sont disposés transversalement par rapport à la direction de transport des œufs à couver (7) en ligne droite et en éventail.
